# EUROPÄISCHE PATENTSCHRIFT

(11) **EP 2 649 937 B1**
(45) Veröffentlichungstag und Bekanntmachung des Hinweises auf die Patenterteilung: **06.08.2014**
(21) Anmeldenummer: 13001313.9
(22) Anmeldetag: 15.03.2013
(51) Int. Cl.: A61B 5/15, A61B 5/154

(54) **Vorrichtung zur Ent- und/oder Aufnahme von Körperflüssigkeiten, insbesondere Blut**
Device for removing and/or receiving body fluids, particularly blood
Dispositif destiné au prélèvement et/ou à la réception de liquides corporels, notamment de sang

(30) Priorität: 11.04.2012 DE 102012007077; 27.12.2012 DE 102012025557
(43) Veröffentlichungstag der Anmeldung: 16.10.2013
(73) Patentinhaber: SARSTEDT AG & Co., 51588 Nümbrecht (DE)
(72) Erfinder: Sarstedt, Walter, 51588 Nümbrecht (DE)
(74) Vertreter: Grosse, Wolf-Dietrich Rüdiger

(56) Entgegenhaltungen:
- EP-A1- 2 277 625
- EP-A2- 0 055 859
- EP-A2- 0 856 280
- WO-A1-03/055392
- CH-A- 303 837
- DE-T2- 3 750 585
- US-A- 4 772 265
- US-A- 5 693 028

## Beschreibung

Die Erfindung betrifft eine Vorrichtung zur Ent- und/oder Aufnahme von Körperflüssigkeiten, insbesondere Blut, umfassend ein Röhrchen, das an seinem oberen, offenen Ende vakuumdicht verschlossen und dort mit einer Kappe versehen ist, die zum Halten einer von einem Nadelhalter getragenen Kanüle bzw. Doppelkanüle vorzugsweise einen domartigen Ansatz aufweist und an ihrem vorderen Ende eine von der Kanüle durchstechbare Membrane aufnimmt.

Blutsammelröhrchen, bei denen das Röhrchen herstellerseitig evakuiert wird, um Blut zu ziehen, sind handelsüblich. Sie müssen derart vakuumdicht sein, dass sie das in der DIN EN 14820 festgelegte Mindestfüllvolumen über die gesamte Haltbarkeit des Produktes hinweg aufnehmen können. Ein nach dem Vakuumprinzip arbeitendes Röhrchen bzw. Behälter kann, wie aus der EP 1 356 302 B1 bekannt, aus Glas, Kunststoff oder anderen gasundurchlässigen und durchsichtigen Materialien bestehen. Zum Befestigen bzw. Aufstecken und Halten eines Kanülenhalters auf einem bzw. einen dort domartigen Ansatz einer Kappe sind durch die EP 1 465 528 B1 verschiedene Varianten von am Außenumfang des domartigen Ansatzes vorgesehenen Verriegelungsmitteln bekannt geworden.

Der Verschluss solcher Vakuumröhrchen erfolgt durch einen dicken Stopfen, in der Regel hergestellt aus Gummi, der in seinem Zentrum eine Materialverdünnung aufweist, welche beim Aufsetzen des Nadelhalters von der Kanüle durchstochen wird.

Ein derartiger in das Röhrchen eingesteckter Verschlussstopfen erfordert besondere Maßnahmen, um einerseits die geforderte Vakuumdichtheit zu gewährleisten und andererseits zu verhindern, dass nach der Blutentnahme beim Abziehen des Nadelhalters bzw. des Röhrchens der Stopfen mit aus dem Röhrchen herausgezogen wird. Außerdem bergen diese Verschlussstopfen das Risiko einer sogenannten Aerosolbildung in sich, d.h., dass beim Entfernen des Stopfens insbesondere mikroskopisch kleine Blutströpfchen verspritzt werden können, die potentiell infektiös wirken und Labormitarbeiter gefährden können.

Der Erfindung liegt daher die Aufgabe zugrunde, eine gattungsgemäße Vorrichtung zu schaffen, die bei einfacher Bauweise eine verbesserte, größere Betriebssicherheit und Bedienungsfreundlichkeit gewährleistet.

Diese Aufgabe wird erfindungsgemäß dadurch gelöst, dass die mit Mitteln zur Verbindung ausgebildete Kappe und das Röhrchen aus einem gasundurchlässigen Material, vorzugsweise PET, bestehen und zwischen der Kappe und dem Röhrchen ein stopfenloses Dichtelement ausgebildet ist.

Es wird damit nicht nur ein stopfenloses Vakuumröhrchen ermöglicht, so dass ein dicker, viel Werkstoff erfordernder Stopfen entfallen kann, sondern die Handhabung wird gleichzeitig deutlich verbessert, weil bei der Entnahme schon gleich die ersten Blutstropfen zu sehen sind. Denn ein den Blutfluss zunächst verdeckender Stopfen muss nicht mehr passiert werden. Schließlich kann praktisch kein Aerosoleffekt auftreten, wenn die Kappe von dem Vakuumröhrchen getrennt wird. Ein Aufschraubvorgang bzw. das Lösen einer hier bevorzugten drehbar geführten Verbindung, wie insbesondere in Form eines Schraubgewindes, d.h. unter Verwendung einer Schraubkappe, optional aber auch als Rastverbindung beispielsweise ein Bajonettverschluss, gewährleistet regelmäßig ein kontrolliertes Öffnungsverhalten.

Ein bevorzugter Vorschlag der Erfindung sieht ein zwischen der Kappe und dem Röhrchen eingeschlossenes, mit einer zentrischen Durchgangsöffnung versehenes ringförmiges Dichtelement vor. Das Dichtelement, insbesondere ein O-Ring oder eine Dichtscheibe, kann sich auf dem bzw. an dem Öffnungsrand des Röhrchens abstützen, wobei es von der aufgeschraubten Kappe fixiert wird. Alternative Dichtelemente können als an der Kappe und/oder dem Röhrchen angeformte konische Abschnitte oder Dichtlippen ausgeführt werden. In jedem Fall liegt eine stopfenlose, vakuumdichte Verbindung vor.

Nach einer bevorzugten Ausführung der Erfindung besteht auch die Kappe aus einem durchsichtigen Material, so dass sich ein von vornherein ohne jegliche Einschränkung einsehbarer Blutfluss erreichen lässt. Als Material für die gasundurchlässige Blutentnahmevorrichtung kommt vorzugsweise PET zum Einsatz. Die für das Röhrchen und die Kappe gleichen Materialien gewährleisten die Gasundurchlässigkeit der Gesamtvorrichtung, und die stopfenlose Dichtelement-Ausbildung erfüllt die Anforderungen an die Vakuumdichtheit.

Bei einem zum Verbinden mit dem Kanülenhalter auf der Schraubkappe ausgebildeten domartigen Ansatz ist, wie bei Schraubkappen von Blutentnahmevorrichtungen nach dem Aspirationssystem an sich bekannt, am oberen, freien Ende eine von der Kanüle bzw. Doppelkanüle durchstechbare, nachgiebige Membrane angeordnet. Die Membrane der erfindungsgemäßen Ausgestaltung besteht übereinstimmend mit den übrigen Teilen aus gasundurchlässigem Material. Vorteilhaft kann sie, um sofort einen Hinweis auf in das Vakuumröhrchen beispielsweise zugegebene Additive zu ermöglichen, bestimmt farbig vorgesehen werden (Farbkodierung). Das Grundmaterial für die Membrane kann Gummi oder dergleichen sein.

In einer vorteilhaften Ausführungsform ist der domartige Ansatz der Kappe an seinem Außenumfang mit Verriegelungsmitteln zum Befestigen einer handelsüblichen Kanüle, wie sie bei nach dem Aspirationsprinzip arbeitenden Blutentnahmesystemen üblich sind, versehen. Durch diese Ausgestaltung wird es dem späteren Anwender ermöglicht, erfindungsgemäße Vakuumblutentnahmeröhrchen und solche, die nach dem Aspirationsprinzip arbeiten, unter Verwendung der gleichen Kanüle, miteinander zu kombinieren.

Weitere Einzelheiten und Merkmale der Erfindung ergeben sich aus den Patentansprüchen und der nachfolgenden Beschreibung eines in der Zeichnung dargestellten Ausführungsbeispiels. Es zeigen:
- Fig. 1: in einem Längsschnitt ein fertig vormontiertes Vakuumröhrchen, das unter Einschluss einer Dichtscheibe als stopfenloses Dichtelement von einer im beschriebenen Ausführungsbeispiel aufgeschraubten, einen domartigen Ansatz aufweisenden Kappe verschlossen wird;
- Fig. 2: das Vakuumröhrchen der Fig. 1 in einer explosiven Darstellung;
- Fig. 3: in einer Gesamtansicht als Einzelheit ein durch eine einen domartigen Ansatz aufweisende Kappe verschlossenes Vakuumröhrchen; und
- Fig. 4: in einer perspektivischen Gesamtansicht ein nach dem Aspirationsprinzip arbeitendes, mit einer Kappe wie in Fig. 3 verschlossenes Blutentnahmeröhrchen und dazu gehörigen Kanülenhalter.

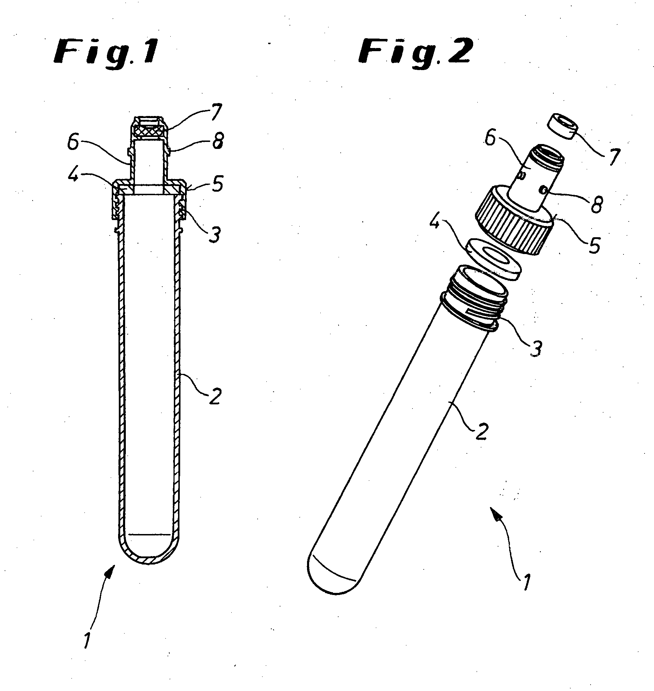

Von einer Blutentnahmevorrichtung 1 ist in den Fig. 1 und 2 als Einzelheit ein Vakuumröhrchen 2 gezeigt, das an seinem oberen, offenen Ende ein Schraubgewinde aufweist und unter Einschluss einer sich auf dem Öffnungsrand des Röhrchens 2 abstützenden Dichtscheibe 4 von einer aufgeschraubten Kappe 5 verschlossen wird. Die Schraubkappe 5 besitzt einen domartigen Ansatz 6 mit an dessen Außenumfang vorgesehenen Verriegelungsmitteln 8 zum Festlegen eines aufgesteckten, hier nicht dargestellten, komplementäre Gegenriegel aufweisenden Nadel- bzw. Kanülenhalters. Am oberen Ende des domartigen Ansatzes 6 ist eine zur Blutentnahme von der Kanüle des Nadelhalters durchstechbare Membrane 7 eingesetzt. Die im Ausführungsbeispiel als stopfenloses Dichtelement vorgesehene Dichtscheibe 4 weist eine zentrische Durchgangsöffnung 9 auf, durch die hindurch das Blut nach dem Durchstechen der Membrane 7 in das Röhrchen bzw. Vakuumröhrchen 2 hinein fließen kann.

Die Schraubkappe 5 einschließlich des domartigen Ansatzes 6 besteht wie das Vakuumröhrchen selbst aus gasundurchlässigem Material, wobei die Vakuumdichtheit des mit Ausnahme des Nadelhalters vormontierten Systems (Fig. 1) durch das Zusammenwirken aller gasundurchlässigen Komponenten erreicht wird. Beim Fertigmontieren wird die Dichtscheibe 4 mit Vorspannung in der Schraubkappe 5 gehalten und beim Aufschrauben der Kappe fest auf den Öffnungsrand des Vakuumröhrchens 2 angedrückt. Die Erkennbarkeit des Blutflusses wird nicht durch einen dicken Stopfen behindert, und bei der Entnahme sind schon die ersten Blutstropfen zu sehen. Das wird dadurch noch weiter unterstützt und verbessert, wenn nicht nur das Vakuumröhrchen 2, sondern weiterhin auch die Schraubkappe 5 aus durchsichtigem Material besteht.

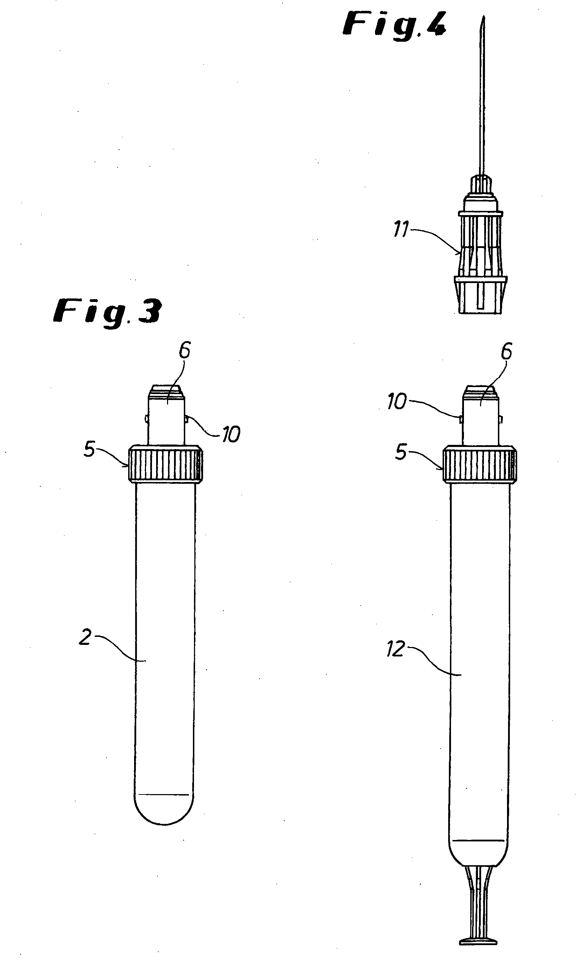

Die Fig. 3 zeigt als Einzelheit ein wie vorbeschrieben vakuumdicht verschlossenes Vakuumröhrchen 2 in einer Gesamtansicht, wobei die Schraubkappe 5 an ihrem domartigen Ansatz 6 mit Verriegelungsmitteln 10 ausgebildet ist, wie sie auch zum Befestigen eines Kanülenhalters 11 bei einem nach dem Aspirationsprinzip arbeitenden, in Fig. 4 dargestellten Blutentnahmeröhrchen 12 üblich sind. Die Zusammenschau der Fig. 3 und 4 verdeutlicht, dass sich sowohl ein Vakuumröhrchen 2 als auch ein zur Aspiration geeignetes Blutentnahmeröhrchen 12 mit einem einheitlichen Kanülenhalter 11 bestücken lässt.

### Bezugszeichenliste:

- 1: Blutentnahmevorrichtung
- 2: Vakuumröhrchen/Röhrchen bzw. Behältnis
- 3: Schraubgewinde
- 4: Dichtscheibe/Dichtelement
- 5: Kappe/Schraubkappe bzw. Verschlusskappe
- 6: domartiger Ansatz
- 7: Membrane
- 8: Verriegelungsmittel
- 9: zentrische Durchgangsöffnung
- 10: Verriegelungsmittel
- 11: Kanülenhalter
- 12: Blutentnahmeröhrchen

## Patentansprüche

1. Vorrichtung zur Ent- und/oder Aufnahme von Körperflüssigkeiten, insbesondere Blut, umfassend ein Röhrchen (2), das an seinem oberen, offenen Ende vakuumdicht verschlossen und dort mit einer Kappe (5) versehen ist, die zum Halten einer von einem Nadelhalter getragenen Kanüle bzw. Doppelkanüle vorzugsweise einen domartigen Ansatz (6) aufweist und an ihrem vorderen Ende eine von der Kanüle durchstechbare Membrane (7) aufnimmt,
**dadurch gekennzeichnet,**
**dass** die mit Mitteln zur Verbindung ausgebildete Kappe (5) und das Röhrchen (2) aus einem gasundurchlässigen Material, vorzugsweise PET, bestehen und zwischen der Kappe (5) und dem Röhrchen (2) ein stopfenloses Dichtelement ausgebildet ist.

2. Vorrichtung nach Anspruch 1,
**gekennzeichnet durch**
ein zwischen der Kappe (5 und dem Röhrchen (2) eingeschlossenes, mit einer zentrischen Durchgangsöffnung (9) versehenes ringförmiges Dichtelement (4).

3. Vorrichtung nach Anspruch 1 oder 2,
**dadurch gekennzeichnet,**
**dass** auch die Kappe (5) aus einem durchsichtigen Material besteht.

4. Vorrichtung nach einem der Ansprüche 1 bis 3,
**dadurch gekennzeichnet,**
**dass** die Mittel zur Verbindung von Kappe (5) und Röhrchen (2) als drehbar geführte Verbindung ausgebildet sind.

5. Vorrichtung nach einem der Ansprüche 1 bis 3,
**dadurch gekennzeichnet,**
**dass** die Mittel zur Verbindung von Kappe (5) und Röhrchen (2) als Rastverbindung ausgebildet sind.

## Claims

1. A device for taking and/or receiving bodily fluids, particularly blood, comprising a small tube (2) that is sealed in a vacuum-tight fashion and provided with a cap (5) on its upper open end, wherein said cap preferably features a dome-like attachment (6) for respectively holding a cannula or dual cannula carried by a needle holder and accommodates a membrane (7) that can be penetrated by the cannula on its front end,
**characterized in**
**that** the cap (5) realized with connecting means and the small tube (2) consist of a gas-impervious material, preferably PET, and a sealing element without stopper is realized between the cap (5) and the small tube (2).

2. The device according to Claim 1, **characterized by** an annular sealing element (4) that is enclosed between the cap (5) and the small tube (2) and provided with a central through-opening (9).

3. The device according Claim 1 or 2, **characterized in that** the cap (5) also consists of a transparent material.

4. The device according to one of Claims 1 to 3, **characterized in that** the means for connecting the cap (5) and the small tube (2) are realized in the form of a rotary coupling.

5. The device according to one of Claims 1 to 3, **characterized in that** the means for connecting the cap (5) and the small tube (2) are realized in the form of a snap-on coupling.

## Revendications

1. Dispositif de prélèvement et/ou d'absorption de liquides physiologiques, en particulier du sang, comprenant un petit tube (2) qui est hermétiquement fermé à son extrémité supérieure ouverte et y est pourvu d'un capuchon (5) qui, pour tenir une canule ou double canule supportée par un porte-aiguille, présente de préférence une pièce bombée rapportée (6) et reçoit à son extrémité avant une membrane (7) pouvant être transpercée par la canule,
**caractérisé en ce que**
le capuchon (5) conformé avec des moyens de raccordement et le petit tube (2) sont faits d'un matériau imperméable au gaz, de préférence du PET, et qu'un élément d'étanchéité sans bouchon est formé entre le capuchon (5) et le petit tube (2).

2. Dispositif selon la revendication 1, **caractérisé par** un élément d'étanchéité de forme annulaire (4) renfermé entre le capuchon (5) et le petit tube (2) et pourvu d'un trou traversant centré (9).

3. Dispositif selon la revendication 1 ou 2, **caractérisé en ce que** le capuchon (5) est aussi fait d'un matériau transparent.

4. Dispositif selon une des revendications 1 à 3, **caractérisé en ce que** les moyens de raccordement du capuchon (5) et du petit tube (2) sont réalisés sous forme d'un raccord guidé en rotation.

5. Dispositif selon une des revendications 1 à 3, **caractérisé en ce que** les moyens de raccordement du capuchon (5) et du petit tube (2) sont réalisés sous forme d'un raccord à déclic.
